# EUROPEAN PATENT SPECIFICATION

(11) **EP 2 245 195 B1**
(45) Date of publication and mention of the grant of the patent: **20.06.2012**
(21) Application number: 09705752.5
(22) Date of filing: 30.01.2009
(51) Int. Cl.: C12Q 1/68, C12Q 1/34, C12N 15/09, C12N 15/10

(54) **TWO STAGE ENRICHMENT OF CELL-FREE FETAL DNA IN MATERNAL PLASMA**
ZWEISTUFIGE ANREICHERUNG VON ZELLFREIER FÖTALER DNA IN MUTTERPLASMA
ENRICHISSEMENT EN DEUX ÉTAPES D'ADN FOETAL ACELLULAIRE DANS DU PLASMA MATERNEL

(30) Priority: 30.01.2008 US 24872 P
(43) Date of publication of application: 03.11.2010
(73) Proprietor: Novartis AG, 4056 Basel (CH)
(72) Inventor: BISCHOFF, Farideh, Sugar Land TX 77479 (US)
(74) Representative: Clegg, Richard Ian
(86) International application number: PCT/US2009/032614
(87) International publication number: WO 2009/097511

(56) References cited:
- HUANG DOROTHY J ET AL: "Improvement of methods for the isolation of cell-free fetal DNA from maternal plasma - Comparison of a manual and an automated method", ANNALS OF THE NEW YORK ACADEMY OF SCIENCES BLACKWELL PUBLISHING, 9600 GARSINGTON RD, OXFORD OX4 2DQ, OXEN, UK SERIES : ANNALS OF THE NEW YORK ACADEMY OF SCIENCES (ISSN 0077-8923(PRINT)), 2006, pages 308-312, XP002616136, & 4TH INTERNATIONAL SYMPOSIUM ON CIRCULATING NUCLEIC ACIDS IN PLASMA/SERUM; LONDON, UK; SEPTEMBER 04 -06, 2005
- HANSON ET AL: "Whole genome amplification strategy for forensic genetic analysis using single or few cell equivalents of genomic DNA", ANALYTICAL BIOCHEMISTRY, ACADEMIC PRESS INC, NEW YORK, vol. 346, no. 2, 15 November 2005 (2005-11-15), pages 246-257, XP005126671, ISSN: 0003-2697, DOI: DOI:10.1016/J.AB.2005.08.017
- JORGEZ CAROLINA J ET AL: "Improving enrichment of circulating fetal DNA for genetic testing: size fractionation followed by whole gene amplification", FETAL DIAGNOSIS AND THERAPY 2009 LNKD- PUBMED:19776594,, vol. 25, no. 3, 22 September 2009 (2009-09-22), pages 314-319, XP002613059,
- DHALLAN R ET AL.: 'Methods to increase the percentage of free fetal DNA recovered from the maternal circulation' JAMA vol. 291, no. 9, 03 March 2004, pages 1114 - 1119, XP009076430
- BISCHOFF F Z ET AL.: 'Cell-free fetal DNA in maternal blood: kinetics, source and structure' HUMAN REPROD UPDATE vol. 11, no. 1, 29 November 2004, pages 59 - 67, XP002413188
- PUSZYK WM ET AL.: 'Noninvasive prenatal diagnosis of aneuploidy using cell-free nucleic acids in maternal blood: promises and unanswered questions' PRENAT DIAGN vol. 28, no. 1, 16 November 2007, pages 1 - 6, XP003026850
- YIN A ET AL.: 'Correlation of maternal plasma total cell-free DNA and fetal DNA levels with short term outcome of first-trimester vaginal bleeding' HUM REPOD vol. 22, no. 6, 07 April 2007, pages 1736 - 1743, XP003026851

## Description

Though fetal DNA is present in maternal plasma, the levels are relatively low to enable routine prenatal genetic analysis. Without being bound to any theory, we believe that a portion of fetal DNA is packaged and thus protected from plasma endo-nucleases. We propose a novel enrichment technique that combines two distinct methods. Separated plasma is first treated with DNase to effectively decrease the percentage of contaminating cell-free maternal DNA fragments. Subsequent increase of fetal sequences is achieved using a modified Whole Genome Amplification (WGA) technique.

**Methods:** Plasma was isolated from maternal whole blood (n=24) then treated with increasing concentrations of DNase. DNase treated DNA was further processed using the Qiagen DNA extraction mini kit prior to a modified WGA protocol to enrich smaller DNA fragments. Presence of fetal sequences was confirmed using real-time Taqman PCR (RT-PCR) to measure β-Globin and DYS1 sequence levels.

**Results:** Fetal DNA sequences were detected following all DNase treatments. Correct detection of male fetuses was achieved in all samples confirmed to have a male fetus (n=10) both before and after WGA. In addition to correct gender determination, the samples (n=7) that were subjected to the highest amount of DNase as well as the modified WGA protocol demonstrated significant enrichment of fetal sequences, attaining 50% fetal DNA.

**Conclusions:** Results confirm that fetal DNA in plasma is protected and resistant to degradation from DNase treatment. These preliminary data also suggest that an optimal level of DNase treatment can be achieved that allows further enrichment using WGA. The observation that DNase eliminates predominantly maternal sequences suggests that cell-free fetal DNA is packaged differently than the maternal counterpart, allowing preferential enrichment of fetal sequences.

### INTRODUCTION

Prenatal genetic diagnosis has relied on invasive procedures such as amniocentesis or chorionic villous sampling (CVS). While these procedures have provided reliable results for many years they still carry a slight risk to the fetus (1). Since the discovery of amplifiable fetal cell-free nucleic acids in maternal plasma (2), there have been numerous studies aimed at determining the potential for clinical non-invasive prenatal genetic tests. While many of these studies have shown great promise, the small existing quantities of fetal DNA has made it difficult to implement clinically. Therefore, we have focused on improving methods of fetal DNA isolation and enrichment. Without being bound to any theory, it is believed that these circulating nucleotides are the result of fetal cells undergoing apoptosis (3). The relative stability of cell-free DNA and RNA in plasma, which is known to contain nucleases, suggests that these nucleic acids are circulating within membrane bound vesicles formed as a result of the mechanism of programmed cell death (4). These fetal DNA fragments are also distinguishable from maternal fragments based on size, fetal fragments generally smaller (<300 bp) than maternal fragments (>500 bp) (5; Jorgez C *et al.,* 2007).
Dhallen et αl (JAMA 2004; 291(9)1114-1119) discloses that addition of formaldehyde to maternal blood samples, coupled with careful processing protocols, increases the relative percentage of free fetal DNA, providing a foundation for development of non-invasive prenatal diagnostic tests to distinguish fetal DNA from maternal DNA in the maternal circulation.
Jorgez and Bischoff (Fetal Diagnosis and Therapy 2009; 25(3): 314-319) discloses that combination of electrophoresis for size separation and WGA led to enriched fetal DNA from plasma.
Huang *et al* (Annals of the New York Academy of Sciences 2006: 308-312) compared manual and automated methods of DNA extraction and discovered that although the manual method yielded more total cell-free DNA, the automated system yielded higher quantities of cell-free DNA of fetal origin. Furthermore, the DNA isolated using the automated system appeared to be of greater purity than that isolated by the manual method, with few inhibitors to downstream real-time PCR reactions.

We describe a novel two stage method for enrichment of fetal fragments. The first stage involves treatment of total maternal plasma (containing both maternal and fetal DNA fragments) with DNase. Given that fetal fragments are more stable and likely packaged by membrane bound apoptotic bodies, we hypothesize that DNase treatment would deplete the overall (unpackaged) maternally derived sequences. The second stage involves a modified whole genome amplification (WGA) protocol designed to amplify smaller fragments, presumably fetal.

### METHODS

Following IRB approval and written informed consent, a total of 24 whole blood samples (10 confirmed male pregnancies, mean gestational age 18 1/7 weeks ranging from 11 4/7 to 25 2/7 weeks; 12 confirmed female pregnancies, mean gestational age 20 1/14 weeks ranging from 9 6/7 to 37 4/7 weeks; and 2 non-pregnant controls, 1 male and 1 female) were collected. For each, approximately 30 ml of blood drawn in ACD vacutainers was processed by an initial centrifugation at 800g for 10min to separate plasma from the cellular fraction. The plasma fraction was removed and centrifuged again at 16,000g for 10 min to further remove any contaminating cellular particles. This fraction was then frozen in 800µl aliquots and later thawed for simultaneous batch processing. Each 800 µl plasma sample was thawed at room temperature then subjected to DNase (Promega, Cat #M6101) treatment at various concentrations (untreated, 1µL, 5µL, 10µL, 30µL of 1 unit/µl). Samples were incubated at 37°C for 1 hour before adding stop solution. Samples were next subjected to DNA extraction using the Qiagen QiAamp Blood Mini Kit (Cat #51106) and eluted in a final volume of 100µL. With slight modifications, the protocol for the GenomePlex^{®} Complete Whole Genome Amplification (WGA) Kit (Sigma, Cat #WGA2-50rxn) was followed on seven maternal blood samples (4 confirmed males, and 3 confirmed females). We modified the procedure by first omitting the manufacturer suggested fragmentation incubation due to the anticipated size of target sequences. Second the cycle number in the amplification step was increased to 20 rather than the suggested 14. Amplified samples were stored at 4°C until RT-PCR analysis for detection and quantification of β-Globin (BGLO354F: GTG CAC CTG ACT CCT GAG GAG A, BGLO455R: CCT TGA TAC CAA CCT GCC CAG) (6) and DYS1 (DYS1F: TCC TGC TTA TCC AAA TTC ACC AT, DYS1R: ACT TCC CTC TGA CAT TAC CTG ATA ATT G) (7) (Applied Biosystems 7700, Foster City, CA). Level of enrichment was determined based on % fetal DNA which was calculated as a ratio of DYS1 to β-Globin. β-Globin represents the total amount of isolated DNA, maternal and fetal, while in the confirmed male pregnancies DYS1 represents the amount of fetal DNA present in the sample.

### RESULTS

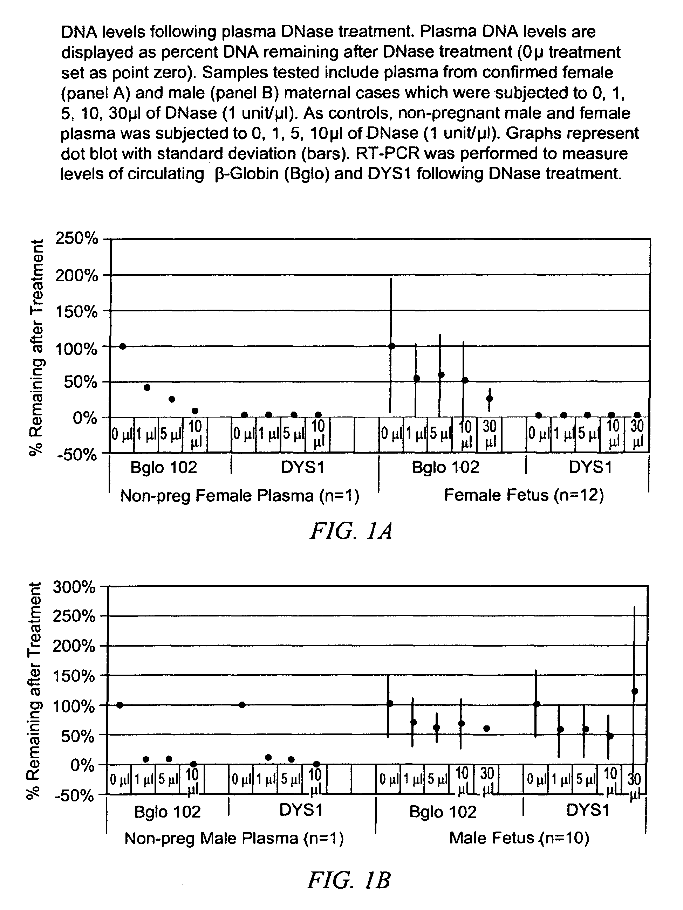

Following initial DNase treatments in control non-pregnant samples, both β-Globin and DYS1 levels decreased as a function of the amount of enzyme added. Each was effectively eliminated by DNase treatment. (Figure 1). In maternal samples, although detected levels of β-Globin were decreased, these levels were persistent despite harsher DNase treatments (916.5 ± 91.2 Geq/ml for male maternal cases and 610.5 ± 389.62 Geq/ml for female maternal cases). No false positive DYS1 levels were detected in any of the known female pregnancies. However, among pregnancies with known male fetuses (n=10), there was 100% detection of DYS1 sequences at all treatment increments (0µl: 127.4 ± 72.3 Geq/ml, 1µl: 71.4 ± 57.3 Geq/ml, 5µl: 71 ± 58.6 Geq/ml, 10µl: 57.1 ± 46.6 Geq/ml, 30µl: 154 ± 179.6 Geq/ml).

WGA was performed on seven DNase treated maternal samples (3 female and 4 male). Though fetal sequences are detected in all samples, increased levels of fetal DYS1 sequences were only observed in the samples that were subjected to 30 µl of DNase (0µl: 1979 ± 2083.9 Geq/ml, 1µl: 1.62 ± 1.6 Geq/ml, 5µl: 723.5 ± 875.7 Geq/ml, 10µl: 0.83 ± 1.22 Geq/ml, 30µl: 7025 ± 4381 Geq/ml). Thus indicating that more stringent DNase treatments were necessary to diminish maternal sequences (based on β-Globin) to the extent that they were not present in levels that out-compete amplification of DYS1 sequences. In the samples that were subjected to 30µl of DNase followed by the modified WGA protocol, we were able to achieve a mean value of 49.96% fetal DNA. In the samples that were not treated with DNase and only subjected to the modified WGA, the mean percent fetal DNA among samples was 11.16%. The samples that were treated with either 1, 5, 10µl of 1unit/µl of DNase all had mean values of 0.01%, 3.5%, and 0.01 % fetal DNA respectively after WGA. Prior to WGA the samples had percentages ranging from 0.05% to 0.17%.

### DISCUSSION

Our results demonstrate that cell-free fetal DNA is resistant to degradation by DNase, supporting the hypothesis that cell-free fetal DNA is packaged in membrane bound vesicles. We effectively removed maternal sequences as well as any contaminating sequences that may lead to false-positive results. The levels of cell-free fetal DNA persist in patient samples versus control samples. This finding confirms that fetal sequences are resistant to degradation and protected or packaged differently than maternal sequences. β-Globin levels represent total DNA (maternal and fetal), thus failure to detect complete digestion of β-Globin is not surprising given that a portion is likely to be fetal. Fetal sequences appear to have a unique molecular characteristic, differentiating it from maternal sequences and enabling enrichment. Detection of all male cases and no false positives suggests that DNase treatment has a novel application in eliminating unwanted maternal sequences that have been degraded further while in circulation.

The GenomePlex^{®} Complete Whole Genome Amplification Kit (Sigma-Aldrich) amplifies genomic DNA by first randomly fragmenting the DNA and then attaching a common sequence on the end which is used to amplify all the fragments by polymerase chain reaction (PCR). We eliminated the fragmentation step from the procedure to prevent larger maternal sequences from fragmenting for subsequent amplification. This potentially provides small pre-existing fragmented fetal sequences an advantage during amplification. WGA increased the fetal to maternal ratio in samples that underwent the most stringent DNase treatments. Prior to WGA, the mean % of fetal DNA was <1% at all treatment levels. However, 50% fetal DNA was achieved in samples that underwent 30 µl of DNase along with the modified WGA protocol, suggesting that this is a viable method of enrichment of cell-free fetal DNA in maternal plasma. Based on β-Globin levels, DNase appears to reduce the quantity of maternal sequences present in the sample, allowing fetal sequences to be amplified. The degradation of maternal nucleic acids prevents competition during amplification of fetal sequences in the PCR reaction. The samples that underwent milder (1, 5, 10µl) DNase treatments displayed lower fetal to maternal ratios after WGA than samples that were not treated with DNAse. One possible explanation is that in these samples the larger maternal sequences were degraded but not completely eliminated, in effect replacing the fragmentation step of the original WGA protocol which allowed more efficient amplification of the maternal sequences.

We describe a combination of methods that allows us to overcome two major complications that has limited non-invasive prenatal DNA genetic testing: low fetal to maternal ratio and small quantity of fetal DNA. Overall, this novel two stage enrichment process shows great potential in selective enrichment followed by amplification.

### ACKNOWLEDGMENTS

NIH grant

### REFERENCES

1. Tabor A, Philip J, Madsen M, Banq J, Obel EB, Nørgaard Pedersen B. Randomised controlled trial of genetic amniocentesis in 4606 low-risk women. Lancet 1986;8493:1287-93.
2. Lo YM, Corbetta N, Chamberlain PF, Rai V, Sargent IL, Redman CW and Wainscoat JS. Presence of fetal DNA in maternal plasma and serum. Lancet 1997;350:485-7.
3. Bischoff FZ, Lewis DE, Simpson JL. Cell-free fetal DNA in maternal blood: kinetics, source and structure. Human Reproductive Update 2004;11:59-67.
4. Orozco AF, Bischoff FZ, Home C, Popek E, Simpson JL, Lewis DE. Hypoxia-induced membrane-bound apoptotic DNA particles: potential mechanism of fetal DNA in maternal plasma. Ann N Y Acad Sci. 2006;1075:57-62.
5. K.C. Allen Chan, Jun Zhang, Angela B.Y. Hui, Nathalie Wong, Tze K. Lau, Tse N. Leung, Kwok-Wai Lo, Dolly W.S. Huang, and Y.M. Dennis Lo. Size Distributions of Maternal and Fetal DNA in Maternal Plasma. Clinical Chemistry 2004;500:88-92.
   Carolina J. Jorgez, Farideh Z. Bischoff. Improving Utility of Circulating DNA for Prenatal Genetic Testing: Fragment Size and Purity. 2007
6. Y M Lo, M S Tein, T K Lau, C J Haines, T N Leung, P M Poon, J S Wainscoat, P J Johnson, A M Chang, and N M Hjelm. Quantitative analysis of fetal DNA in maternal plasma and serum: implications for noninvasive prenatal diagnosis. Am J Hum Genet. 1998; 62:768-75.
7. Tuangsit Wataganara, Erik LeShane, Antonio Farina, Geralyn M. Messerlian, Thomas Lee, Jacob A. Canick, Diana W. Bianchi. Maternal Serum Cell-Free DNA Levels are Increased in Cases of Trisomy 13 but not 18. Hum Genet 2003;112:204-8

## Claims

1. A method for enriching fetal nucleic acid comprising treating a biological sample of a maternal host containing cell-free fetal nucleic acid with a composition comprising an agent with DNase activity, wherein a first percentage of fetal nucleic acid in the biological sample prior to the treatment is lower than a second percentage of fetal nucleic acid in the biological sample after the treatment and wherein a first percentage of maternal nucleic acid in the biological sample prior to the treatment is higher than a second percentage of maternal nucleic acid in the biological sample after the treatment.

2. The method of claim 1, wherein the biological sample is a blood sample of a maternal host.

3. The method of claim 1, wherein the biological sample is a plasma or serum sample of a maternal host.

4. The method of claim 1, wherein the agent with DNase activity is DNase.

5. The method of claim 1, wherein the agent with DNase activity is DNase and the amount of DNase is from about 10 to 200 unit/µl

6. The method of claim 1, wherein the second percentage is from about 10% to 50%.

7. The method of claim 1, wherein the second percentage is at least 10%, 20%, 30%, 40%, or 50%.

8. The method of claim 1 further comprising amplifying fetal nucleic acid in the biological sample after the treatment.

9. The method of claim 1 further comprising amplifying fetal nucleic acid in the biological sample after the treatment via a Whole Genome Amplification (WGA) method.

10. The method of claim 9, wherein the WGA method is conducted without fragmentation incubation.

## Patentansprüche

1. Verfahren zur Anreicherung von fötaler Nucleinsäure, umfassend das Behandeln einer biologischen Probe von einem maternalen Wirt, die zellfreie fötale Nucleinsäure enthält, mit einer Zusammensetzung, die ein Mittel mit DNase-Aktivität umfasst, wobei ein erster Prozentsatz an fötaler Nucleinsäure in der biologischen vor der Behandlung niedriger ist als ein zweiter Prozentsatz an fötaler Nucleinsäure in der biologischen Probe nach der Behandlung und wobei ein erster Prozentsatz an maternaler Nucleinsäure in der biologischen Probe vor der Behandlung höher ist als ein zweiter Prozentsatz von maternaler Nucleinsäure in der biologischen Probe nach der Behandlung.

2. Verfahren nach Anspruch 1, wobei die biologische Probe eine Blutprobe von einem maternalen Wirt ist.

3. Verfahren nach Anspruch 1, wobei die biologische Probe eine Plasma- oder Serumprobe von einem maternalen Wirt ist.

4. Verfahren nach Anspruch 1, wobei das Mittel mit DNase-Aktivität DNase ist.

5. Verfahren nach Anspruch 1, wobei das Mittel mit DNase-Aktivität DNase ist und die Menge an DNase etwa 10 bis 200 Einheiten/µl beträgt.

6. Verfahren nach Anspruch 1, wobei der zweite Prozentsatz etwa 10 % bis 50 % beträgt.

7. Verfahren nach Anspruch 1, wobei der zweite Prozentsatz zumindest 10 %, 20 %, 30 %, 40 % oder 50 % beträgt.

8. Verfahren nach Anspruch 1, das weiters das Amplifizieren von fötaler Nucleinsäure in der biologischen Probe nach der Behandlung umfasst.

9. Verfahren nach Anspruch 1, das weiters das Amplifizieren von fötaler Nucleinsäure in der biologischen Probe nach der Behandlung mittels eines Gesamtgenomamplifikations- (WGA-) Verfahrens umfasst.

10. Verfahren nach Anspruch 9, wobei das VGA-Verfahren ohne Fragmentationsinkubation erfolgt.

## Revendications

1. Méthode d'enrichissement d'un acide nucléaire foetal comprenant le traitement d'un échantillon biologique d'un hôte maternel contenant l'acide nucléique foetal exempt de cellules avec une composition comprenant un agent avec une activité de DNase, où un premier pourcentage d'acide nucléique foetal dans l'échantillon biologique avant le traitement est plus bas qu'un deuxième pourcentage d'acide nucléique foetal dans l'échantillon biologique après le traitement, et où un premier pourcentage d'acide nucléique maternel dans l'échantillon biologique avant le traitement est plus élevé qu'un deuxième pourcentage d'acide nucléique maternel dans l'échantillon biologique après le traitement.

2. Méthode selon la revendication 1, où l'échantillon biologique est un échantillon de sang d'un hôte maternel.

3. Méthode selon la revendication 1, où l'échantillon biologique est un échantillon de plasma ou de sérum d'un hôte maternel.

4. Méthode selon la revendication 1, où l'agent avec l'activité de DNase est la DNase.

5. Méthode selon la revendication 1, où l'agent avec l'activité de DNase est la DNase et la quantité de DNase est d'environ 10 à 200 unités/µl.

6. Méthode selon la revendication 1, où le deuxième pourcentage est d'environ 10% à 50%.

7. Méthode selon la revendication 1, où le deuxième pourcentage est au moins de 10%, 20%, 30%, 40% ou 50%.

8. Méthode selon la revendication 1, comprenant en outre l'amplification de l'acide nucléique foetal dans l'échantillon biologique après le traitement.

9. Méthode selon la revendication 1, comprenant en outre l'amplification de l'acide nucléique foetal dans l'échantillon biologique après le traitement par une méthode d'Amplification Génomique Totale (WGA).

10. Méthode selon la revendication 9, où la méthode WGA est exécutée sans incubation de fragmentation.
